(19) Europäisches Patentamt
European Patent Office
Office européen des brevets

(11) EP 2 533 781 B1

(12) **EUROPEAN PATENT SPECIFICATION**

(45) Date of publication and mention of the grant of the patent:
**16.11.2016 Bulletin 2016/46**

(21) Application number: **11742929.0**

(22) Date of filing: **12.02.2011**

(51) Int Cl.:
***A61K 31/47*** *(2006.01)* ***A61K 31/555*** *(2006.01)*
***A61K 33/24*** *(2006.01)* ***A61P 35/00*** *(2006.01)*
***A61P 35/02*** *(2006.01)*

(86) International application number:
**PCT/US2011/024662**

(87) International publication number:
**WO 2011/100642 (18.08.2011 Gazette 2011/33)**

# (54) METHOD FOR TREATING HEMATOLOGICAL CANCERS OF MYELOID ORIGIN

VERFAHREN ZUR BEHANDLUNG VON BLUTKREBS MYELOISCHEN URSPRUNGS

PROCÉDÉ DE TRAITEMENT DE CANCERS HÉMATOLOGIQUES D'ORIGINE MYÉLOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2010 US 304244 P**

(43) Date of publication of application:
**19.12.2012 Bulletin 2012/51**

(73) Proprietor: **Niiki Pharma Inc.**
**Hoboken, NJ 07030 (US)**

(72) Inventors:
- **SHESHBARADARAN, Hooshmand**
  **Hoboken, New Jersey 07030 (US)**
- **PROKOP, Aram**
  **D-50924 Cologne (DE)**
- **LEE, Soo-Young**
  **D-50924 Cologne (DE)**
- **BAERGA, Rebecca**
  **Hoboken, New Jersey 07030 (US)**
- **COBB, Jenel**
  **Hoboken, New Jersey 07030 (US)**
- **VALIAHDI, Seied, Mojtaba**
  **A-1090 Vienna (AT)**
- **KEPPLER, Bernhard**
  **A-1090 Vienna (AT)**

(74) Representative: **Tostmann, Holger Carl**
**Wallinger Ricker Schlotter Tostmann**
**Patent- und Rechtsanwälte**
**Zweibrückenstrasse 5-7**
**80331 München (DE)**

(56) References cited:
**WO-A1-2006/110931** **WO-A1-2010/114861**
**US-A- 5 525 598** **US-A1- 2007 231 407**
**US-A1- 2009 137 620** **US-A1- 2009 208 591**

- **BERGGREN MARGARETA M ET AL: "Inhibition of protein tyrosine phosphatase by the antitumor agent gallium nitrate", CANCER RESEARCH, vol. 53, no. 8, 1993, pages 1862-1866, XP002705007, ISSN: 0008-5472**
- **ANDRIES R. JONKHOFF ET AL: "Radiotoxicity of 67-Gallium on myeloid leukemic blasts", LEUKEMIA RESEARCH, vol. 19, no. 3, 1 March 1995 (1995-03-01), pages 169-174, XP055072369, ISSN: 0145-2126, DOI: 10.1016/0145-2126(94)00130-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 533 781 B1

## Description

Field of the Invention

The present invention generally relates to pharmaceutical compositions and compounds for use in treating hematological cancer of myeloid origin, and particularly to a pharmaceutical composition having tris(8-quinolinolato) gallium(III) for use in treating hematological cancer of myeloid origin.

Background of the Invention

Hematological malignancies or blood cancers are a diverse but related cancers originated from bone marrow or lymphatic tissues, affecting blood functions. Each year, new cases of leukemia, Hodgkin and non-Hodgkin lymphoma and myeloma account for almost 10 percent of all new cancer cases diagnosed in the United States. While targeted therapies using antibodies and kinase inhibitors (e.g., imatinib - a BCR-ABL inhibitor) have been developed, chemotherapy and radiation therapy are still heavily relied upon in the management of blood cancers. They typically exhibit significant side effect and produce low efficacy. There is a need for new classes of drugs with distinct mechanism of actions in treating blood cancers. US Patent Publication No. 2009/0137620 discloses that the compound tris(8-quinolinolato)gallium(III) has been shown to be particularly effective in causing apoptosis and cell death in melanoma cell lines. However, it is unknown whether the compound is useful in treating blood cancers, especially those blood cancers refractory to other anti-cancer drugs.

Jonkhoff AR et al., Leuk Res. 1995;19(3):169-174 reports *in vitro* data about the radiotoxicity of $^{67}Ga$ on isolated peripheral blast cells from patients with acute myelogenous leukemia (AML) and concludes that $^{67}Ga$ induces clonogenic cell death in leukemic blasts.

Summary of the Invention

The present invention provides the following embodiments:

1. A compound of Formula (I)

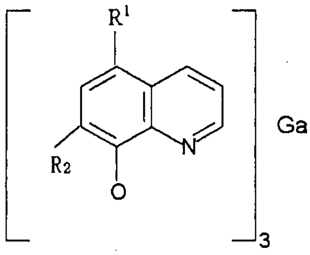

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of hematological cancer, wherein said hematological cancer is of myeloid origin.

2. Use of a compound as defined in embodiment 1 or a pharmaceutically acceptable salt thereof for the manufacturing of a medicament for treating hematological cancer, wherein said hematological cancer is of myeloid origin.

3. The compound for use of embodiment 1 or the use of embodiment 2, wherein said compound is tris(8-quinolinolato)gallium(III).

4. The compound for use of embodiment I or 3 or the use of embodiment 2 or 3, wherein said hematological cancer is myelogenous leukemia.

5. The compound for use of any one of embodiments 1, 3 and 4 or the use of any one of embodiments 2 to 4, wherein said hematological cancer is not acute promyelocytic leukemia.

6. The compound for use of any one of embodiments 1 and 3 to 5 or the use of any one of embodiments 2 to 5, wherein said hematological cancer is selected from acute myeloblastic leukemia, acute megakaryoblastic leukemia,

chronic myelogenous leukemia, acute monocytic leukemia, myelodysplastic syndromes (MDS), or myeloproliferative diseases.

7. The compound for use of any one of embodiments 1 and 3 to 6 or the use of any one of embodiments 2 to 6, wherein said hematological cancer is a refractory hematological cancer.

8. The compound for use of embodiment 7 or the use of embodiment 7, wherein said hematological cancer is refractory to a treatment comprising one or more drugs selected from the group consisting of vinca alkaloids, anthracyclines, anthracenediones, epipodophyllotoxins, camptothecins, lenalidomide, thalidomide, cytarabine and fludarabine.

9. The compound for use of embodiment 7 or the use of embodiment 7, wherein said hematological cancer is previously treated with a camptothecin drug (e.g., topotecan).

10. The compound for use of embodiment 7 or the use of embodiment 7, wherein said hematological cancer is chronic myelogenous leukemia (CML) previously treated with a PDGF-Rβ inhibitor (e.g., imatinib).

11. The compound for use of embodiment 7 or the use of embodiment 7, wherein said hematological cancer is acute myeloid leukemia previously treated with cytarabine.

12. The compound for use of embodiment 7 or the use of embodiment 7, wherein said hematological cancer is acute myeloid leukemia (AML) previously treated with fludarabine.

13. The compound for use of embodiment 7, wherein said hematological cancer is MDS previously treated with lenalidomide.

In one aspect, the present invention provides a compound according to Formula (I) below or a pharmaceutically acceptable salt thereof (e.g., tris(8-quinolinolato)gallium(III)) for use in treating a hematological cancer of myeloid origin, particularly causing apoptosis and cell death in hematological tumor cells of a patient, comprising administering to the patient having hematological cancer of myeloid origin a therapeutically effective amount of said compound.

In accordance with another aspect, a compound according to Formula (I) below or a pharmaceutically acceptable salt thereof (e.g., tris(8-quinolinolato)gallium(III)) for use in treating a refractory hematological cancer of myeloid origin is provided comprising administering a therapeutically effective amount of the compound according to Formula (I) or a pharmaceutically acceptable salt thereof (e.g., tris(8-quinolinolato)gallium(III)) to a patient refractory to one or more drugs chosen from vinca alkaloids, anthracyclines (e.g., doxorubicin), anthracenediones, epipodophyllotoxins, camptothecins, lenalidomide, thalidomide, methotrexate, cyclophosphamide, Adriamycin, prednisone, cytarabine , Ara-C, and fludarabine.

Use of the compound according to Formula (I) below or a pharmaceutically acceptable salt thereof (e.g., tris(8-quinolinolato)gallium(III)) for the manufacture of a medicament for use as defined in the claims is also provided.

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

<u>Brief Description of Drawings</u>

**Figure 1** is a graph showing cell viability and inhibition of proliferation of BJAB lymphoma cells by tris(8-quinolinolato)gallium(III), which inhibits proliferation in a dose-dependent manner up to 100% (exposure time 24 hours);
**Figure 2** is a graph showing the impairment of mitochondrial membrane potential measured by flow cytometric analysis of BJAB cells after 48 hours of incubation with different concentration of tris(8-quinolinolato)gallium(III) and staining the cells with the cationic dye JC-1 (5,5',6,6'-tetrachloro-1,1,3,3'-tetraethylbenzimidazolylcarbocyanine iodide). Values of the mitochondrial permeability transition are given as percentages of cells with low $\Delta\Psi m \pm$ SD (n=3);
**Figure 3** is a diagram showing apoptosis induction measured via flow cytometric determination of hypodiploid DNA in BJAB cells after treatment with tris(8-quinolinolato)gallium(III) for 72 hours. Data are given in % hypodiploidy (subG1) $\pm$ ESD (n=3), which is consistent with the number of apoptotic cells;
**Figure 4A and 4B** are graphs apoptosis induction by tris(8-quinolinolato)gallium(III) in vincristine (Figure 4A) and daunorubicin (Figure 4B) resistant Nalm-6 cells. After 72 hours of incubation with different concentrations of the

agent, DNA fragmentation was measured via FACS scan analysis. The results show clearly that tris(8-quinolinolato)gallium(III) is capable to overcome resistance to the conventional drugs vincristine and daunorubicin. Values of DNA fragmentation are given as percentages of cells with hypodiploid DNA ± s.d. (n=3);

**Figure 5** shows DNA fragmentation measured by flow cytometric analysis after treatment of vector-transfected (BJAB mock) or FADD-dn-transfected BJAB cells (BJAB FADDdn) with different concentrations of tris(8-quinolinolato)gallium(III) for 72 hours. Values of DNA fragmentation are given as percentages of cells with hypodiploid DNA ± s.d. (n=3);

**Figure 6** includes chromatograms showing Western blot analysis of tris(8-quinolinolato)gallium(III)-treated BJAB cells. Epirubicin was used as a positive control. After incubation for 24 hours the protein extracts were fractionated on a denaturating 4-20% polyacrylamide gel, transferred to nitrocellulose and detected with anti-caspase-3/-9 and anti-ß-actin antibody. **A:** caspase-9 (procaspase: 47 kDa; cleavage product: 37 kDa), **B:** caspase-3 (procaspase: 32 kDa; cleavage products: 18 and 17 kDa), C: ß-actin (42 kDa);

**Figure 7** is a graph showing the dose-dependent growth inhibition by tris(8-quinolinolato)gallium(III) (MTT assay) in DoHH2 cells. X axis: tris(8-quinolinolato)gallium(III) concentration (μM), Y axis: % control;

**Figure 8** is a graph showing the dose-dependent growth inhibition by tris(8-quinolinolato)gallium(III) (MTT assay) in Granta 519 cells. X axis: tris(8-quinolinolato)gallium(III) concentration (μM), Y axis: % control; and

**Figure 9** is a graph showing the dose-dependent growth inhibition by tris(8-quinolinolato)gallium(III) (MTT assay) in WSU-DLCL2 cells. X axis: tris(8-quinolinolato)gallium(III) concentration (μM), Y axis: % control.

## Detailed Description of the Invention

The present invention is at least in part based on the discovery that the compound tris(8-quinolinolato)gallium(III) is especially effective in treating various hematological cancers including leukemia and lymphoma. Accordingly, in accordance with a first aspect of the present invention, a compound according to Formula (I) below or a pharmaceutically acceptable salt thereof is provided for use in treating hematological cancer of myeloid origin, which comprises treating a patient having a hematological cancer of myeloid origin in need of treatment with a therapeutically effective amount of a gallium complex of Formula (I)

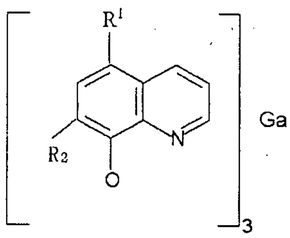

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof. In one embodiment, the hematological cancer is not acute promyelocytic leukemia. That is, the present invention is directed to the use of an effective amount of a compound according to Formula (I) or a pharmaceutically acceptable salt thereof for the manufacture of medicaments for treating a hematological cancer of myeloid cancer in patients identified or diagnosed as having a hematological cancer of myeloid origin, preferably said hematological cancer not being acute promyelocytic leukemia. In preferred embodiments, the gallium complex is tris(8-quinolinolato)gallium(III) or a pharmaceutically acceptable salt thereof.

Hematological malignancies are typically derived from either of the two major blood cell lineages: myeloid and lymphoid cells. Lymphomas and lymphocytic or lymphoblastic leukemias are derived from lymphoid cells. These include acute lymphocytic leukemia (ALL), chronic lymphocytic leukemia (CLL), hairy cell leukemia (HCL), T-cell or B-cell prolymphocytic leukemia (T-PLL or B-PLL) and myelomas. Myeloid or myelogenous leukemias, myelodysplastic syndromes (MDS), and myeloproliferative diseases (MPD) are derived from myeloid cells. These include acute myelogenous leukemia (AML), chronic myelogenous leukemia (CML), chronic granulocytic leukemia (CGL), acute monoblastic/monocytic leukemia (AMOL) and myelofibrosis.

Different experimental models have been tested with the compound tris(8-quinolinolato)gallium(III) and it has been surprisingly discovered that the compound has a broad spectrum of activity against different kinds of hematological cancers.

The hematological cancer treated in accordance with the present invention is a hematological cancer of myeloid origin, i.e., derived from myeloid cells, preferably said hematological cancer not being acute promyelocytic leukemia. In

specific embodiments, the compound of the present invention is used for treating a myelogenous leukemia. In some specific embodiments, the compound of the present invention is used for treating a myelogenous leukemia, wherein the myelogenous leukemia is not acute promyelocytic leukemia. In some specific embodiments the compound of the present invention is used for treating acute myelogenous leukemia (AML), chronic granulocytic leukemia (CGL), acute monoblastic/monocytic leukemia (AMOL), chronic myelogenous leukemia (CML), myelodysplastic syndrome (MDS), myeloproliferative disease (MPD), and myelofibrosis, preferably said hematological cancer not being acute promyelocytic leukemia.

In the various embodiments of this aspect of the present invention, the treatment optionally also comprises a step of diagnosing or identifying a patient as having any one of a hematological cancer of myeloid origin. The identified patient is then treated with or administered with a therapeutically effective amount of a compound of the present invention, e.g., tris(8-quinolinolato)gallium(III). Various hematological cancers can be diagnosed in any conventional diagnostic methods known in the art including complete blood count, blood film, lymph node biopsy, bone marrow biopsy, cytogenetics analysis (e.g., for AML, CML), or immuophenotyping (e.g., for lymphoma, myeloma, CLL).

In addition, it has also been surprisingly discovered that the compound tris(8-quinolinolato)gallium(III) is equally effective in hematological cancer cells resistant to one or more drugs including vinca alkaloids, anthracyclines, anthracenediones, epipodophyllotoxins, camptothecins, lenalidomide, thalidomide, methotrexate, cytarabine, fludarabine, cyclophosphamide, adriamycin, and prednisone. Accordingly, another aspect of the present invention provides a compound of Formula (I) or a pharmaceutically acceptable salt thereof for use in treating refractory hematological cancer of myeloid origin comprising treating a patient identified as having refractory hematological cancer of myeloid origin with a therapeutically effective amount of the compound of Formula (I) or a pharmaceutically acceptable salt thereof (e.g., tris(8-quinolinolato)gallium(III). In one embodiment, the patient has a hematological cancer of myeloid origin. that is refractory to a treatment comprising one or more drugs selected from the group consisting of vinca alkaloids, anthracyclines, anthracenediones, epipodophyllotoxins, camptothecins, lenalidomide, thalidomide, methotrexate, cytarabine, fludarabine, cyclophosphamide, adriamycin, vincristine, and prednisone. That is, the present invention is also directed to the use of a compound of Formula (I) or a pharmaceutically acceptable salt thereof (e.g., tris(8-quinolinolato)gallium(III)) for the manufacture of medicaments for treating refractory hematological cancer of myeloid origin, e.g., a hematological cancer of myeloid origin refractory to one or more drugs chosen from vinca alkaloids (e.g., vincristine, vinblastine, vinorelbine), anthracyclines (e.g., doxorubincin, daunorubicin, epirubicin), anthracenediones (e.g., mitoxantrone and pixantrone), epipodophyllotoxins (e.g., etoposide and teniposide), camptothecins (e.g., topotecan, irinotecan), lenalidomide, thalidomide, methotrexate, Ara-C (cytarabine), fludarabine, cyclophosphamide, adriamycin, vincristine, prednisone, and taxane (e.g., paclitaxel and docetaxel).

The term "refractory hematological cancer," as used herein refers to a hematological cancer that either fails to respond favorably to an anti-neoplastic treatment that does not include a compound of Formula (I), or alternatively, recurs or relapses after responding favorably to an antineoplastic treatment that does not include a compound of Formula (I). Accordingly, "a hematological cancer refractory to a treatment" as used herein means a hematological cancer that fails to respond favorably to, or resistant to, the treatment, or alternatively, recurs or relapses after responding favorably to the treatment.

Thus, in some embodiments, of the present invention, a compound of Formula (I) or a pharmaceutically acceptable salt thereof (e.g., tris(8-quinolinolato)gallium(III)) is used to treat hematological cancer of myeloid origin patients having a tumor that exhibits resistance to a treatment comprising one or more drugs selected from the group consisting of from vinca alkaloids (e.g., vincristine, vinblastine, vinorelbine), anthracyclines (e.g., doxorubicin, daunorubicin, epirubicin), anthracenediones (e.g., mitoxantrone and pixantrone), epipodophyllotoxins (e.g., etoposide and teniposide), camptothecins (e.g., topotecan, irinotecan), lenalidomide, thalidomide, methotrexate, Ara-C (cytarabine), fludarabine, cyclophosphamide, adriamycin, vincristine, prednisone, and taxane (e.g., paclitaxel and docetaxel). In other words, the compound is used to treat a hematological cancer patient having previously been treated with a treatment regimen that includes one or more drugs selected from the group consisting of from vinca alkaloids (e.g., vincristine, vinblastine, vinorelbine), anthracyclines (e.g., doxorubincin, daunorubicin, epirubicin), anthracenediones (e.g., mitoxantrone and pixantrone), epipodophyllotoxins (e.g., etoposide and teniposide), camptothecins (e.g., topotecan, irinotecan), lenalidomide, thalidomide, methotrexate, Ara-C (cytarabine), fludarabine, cyclophosphamide, adriamycin, vincristine, prednisone, and taxane (e.g., paclitaxel and docetaxel), and whose hematological cancer was found to be non-responsive to the treatment regimen or have developed resistance to the treatment regimen. In other embodiments, the compound is used to treat a hematological cancer of myeloid origin patient previously treated with a treatment comprising one or more drugs selected from the group consisting of vinca alkaloids (e.g., vincristine, vinblastine, vinorelbine), anthracyclines (e.g., doxorubincin, daunorubicin, epirubicin), anthracenediones (e.g., mitoxantrone and pixantrone), epipodophyllotoxins (e.g., etoposide and teniposide), camptothecins (e.g., topotecan, irinotecan), lenalidomide, thalidomide, methotrexate, Ara-C (cytarabine), fludarabine, cyclosphosphamide, adriamycin, vincristine, prednisone, and taxane (e.g., paclitaxel and docetaxel), but the hematological cancer of myeloid origin has recurred or relapsed, that is, a hematological cancer of myeloid origin patient who has previously been treated with one or more such drugs, and whose cancer was initially

responsive to the previously administered one or more such drugs, but was subsequently found to have relapsed.

In specific embodiments, a compound of Formula (I) or a pharmaceutically acceptable salt thereof (e.g., tris(8-quinolinolato)gallium(III)) is used to treat hematological cancer of myeloid origin patients previously treated with a vinca alkaloid (e.g., vincristine, vinblastine, or vinorelbine), e.g., who have a tumor that exhibits resistance to, or relapsed after, a treatment including, a vinca alkaloid (e.g., vincristine, vinblastine, or vinorelbine).

In yet other specific embodiments, a compound of Formula (I) or a pharmaceutically acceptable salt thereof (e.g., tris(8-quinolinolato)gallium(III)) is used to treat hematological cancer of myeloid origin patients previously treated with an anthracycline (e.g., doxorubicin, daunorubicin, idarubicin or epirubicin), e.g., who have a hematological cancer of myeloid origin that exhibits resistance to, or relapsed after, a treatment including, an anthracycline (e.g., doxorubicin, daunorubicin, idarubicin or epirubicin).

In still other specific embodiments, a compound of Formula (I) or a pharmaceutically acceptable salt thereof (e.g., tris(8-quinolinolato)gallium(III)) is used to treat hematological cancer of myeloid origin patients previously treated with anthracenedione (e.g., mitoxantrone or pixantrone), e.g., who have a hematological cancer with myeloid origin that exhibits resistance to, or relapsed after, a treatment including, mitoxantrone or pixantrone.

In still other specific embodiments, a compound of Formula (I) or a pharmaceutically acceptable salt thereof (e.g., tris(8-quinolinolato)gallium(III)) is used to treat hematological cancer of myeloid origin patients previously treated with a camptothecin drug (e.g., topotecan, irinotecan), e.g., who have a hematological cancer of myeloid origin that exhibits resistance to, or relapsed after, a treatment including, topotecan or irinotecan.

In still other specific embodiments, a compound of Formula (I) or a pharmaceutically acceptable salt thereof (e.g., tris(8-quinolinolato)gallium(III)) is used to treat hematological cancer of myeloid origin (e.g., chronic myelogenous leukemia (CML)) patients previously treated with PDGF-Rβ inhibitor (e.g., imatinib), e.g., who have a hematological cancer of myeloid origin (e.g., chronic myelogenous leukemia (CML)) that exhibits resistance to, or relapsed after, a treatment including imatinib.

In other specific embodiments, a compound of Formula (I) or a pharmaceutically acceptable salt thereof (e.g., tris(8-quinolinolato)gallium(III)) is used to treat hematological cancer of myeloid origin patients previously treated with lenalidomide or thalidomide, e.g., who have hematological cancer of myeloid origin that exhibits resistance to, or relapsed after, a treatment including lenalidomide or thalidomide.

In other specific embodiments, a compound of Formula (I) or a pharmaceutically acceptable salt thereof (e.g., tris(8-quinolinolato)gallium(III)) is used to treat hematological cancer of myeloid origin (e.g., myelofibrosis) patients previously treated with lenalidomide or thalidomide, e.g., who have hematological cancer of myeloid origin (e.g., myelofibrosis) that exhibits resistance to, or relapsed after, a treatment including lenalidomide or thalidomide.

In other specific embodiments, a compound of Formula (I) or a pharmaceutically acceptable salt thereof (e.g., tris(8-quinolinolato)gallium(III)) is used to treat hematological cancer of myeloid origin patients (e.g., patients having acute myeloid leukemia) previously treated with cytarabine, e.g., who have hematological cancer of myeloid origin (e.g., acute myeloid leukemia) that exhibits resistance to, or relapsed after, a treatment including cytarabine.

In other specific embodiments, a compound of Formula (I) or a pharmaceutically acceptable salt thereof (e.g., tris(8-quinolinolato)gallium(III)) is used to treat hematological cancer of myeloid origin patients previously treated with methotrexate, e.g., who have hematological cancer of myeloid origin that exhibits resistance to, or relapsed after, a treatment including methotrexate.

In other specific embodiments, a compound of Formula (I) or a pharmaceutically acceptable salt thereof (e.g., tris(8-quinolinolato)gallium(III)) is used to treat hematological cancer of myeloid origin patients (e.g., patients having acute myeloid leukemia (AML)) previously treated with fludarabine, e.g., who have hematological cancer of myeloid origin (e.g., acute myeloid leukemia (AML)) that exhibits resistance to, or relapsed after, a treatment including fludarabine.

In yet other specific embodiments, a compound of Formula (I) or a pharmaceutically acceptable salt thereof (e.g., tris(8-quinolinolato)gallium(III)) is used to treat hematological cancer of myeloid origin patients (e.g., patients having myelodysplastic syndrome (MDS)) previously treated with azacitidine or decitabine, e.g., who have hematological cancer of myeloid origin (e.g., myelodysplastic syndrome (MDS)) that exhibits resistance to, or relapsed after, a treatment including lenalidomide, azacitidine or decitabine.

In yet other specific embodiments, a compound of Formula (I) or a pharmaceutically acceptable salt thereof (e.g., tris(8-quinolinolato)gallium(III)) is used to treat hematological cancer of myeloid origin patients previously treated with one or more drugs selected from the group of cyclophosphamide, adriamycin, vincristine, and prednisone, e.g., who have hematological cancer of myeloid origin that exhibits resistance to, or relapsed after, a treatment one or more drugs selected from the group of cyclophosphamide, adriamycin, vincristine, and prednisone (e.g., CHOP regimen).

To detect a refractory hematological cancer, patients undergoing initial treatment can be carefully monitored for signs of resistance, non-responsiveness or recurring hematological cancer. This can be accomplished by monitoring the patient's cancer's response to the initial treatment which, e.g., may include one or more drugs selected from the

group consisting of vinca alkaloids, anthracyclines, anthracenediones, epipodophyllotoxins, camptothecins, lenalidomide, thalidomide, cytarabine and fludarabine, cyclophosphamide, adriamycin, vincristine, and prednisone. The response, lack of response, or relapse of the cancer to the initial treatment can be determined by any suitable method practiced in the art. For example, this can be accomplished by the assessment of tumor size and number. An increase in tumor size or, alternatively, tumor number, indicates that the tumor is not responding to the chemotherapy, or that a relapse has occurred. The determination can be done according to the "RECIST" criteria as described in detail in Therasse et al, J. Natl. Cancer Inst. 92:205-216 (2000).

A method is further disclosed herein for preventing or delaying the onset of hematological cancer, or preventing or delaying the recurrence of hematological cancer, which comprises treating a patient in need of the prevention or delay with a prophylactically effective amount of a compound of Formula (I) or a pharmaceutically acceptable salt thereof (e.g., tris(8-quinolinolato)gallium(III)).

For purposes of preventing or delaying the recurrence of hematological cancer, hematological cancer patients who have been treated and are in remission or in a stable or progression free state may be treated with a prophylactically effective amount of a compound of Formula (I) or a pharmaceutically acceptable salt thereof (e.g., tris(8-quinolinolato)gallium(III)) to effectively prevent or delay the recurrence or relapse of hematological cancer.

As used herein, the phrase "treating ... with ..." or a paraphrase thereof means administering a compound to the patient or causing the formation of a compound inside the body of the patient.

In accordance with the present invention, hematological cancer of myeloid origin can be treated with a therapeutically effective amount of a compound of Formula (I) or a pharmaceutically acceptable salt thereof (e.g., tris(8-quinolinolato)gallium(III)) alone as a single agent, or alternatively in combination with one or more other anti-cancer agents. Example of pharmaceutically acceptable salts include alkali metal salts (e.g., sodium or potassium salt), ammonium salts, etc.

The pharmaceutical compounds of Formula (I) can be administered through intravenous injection or oral administration or any other suitable means at an amount of from 0.1 mg to 1000 mg per kg of body weight of the patient based on total body weight. The active ingredients may be administered at predetermined intervals of time, e.g., three times a day. It should be understood that the dosage ranges set forth above are exemplary only and are not intended to limit the scope of this invention. The therapeutically effective amount of the active compound can vary with factors including, but not limited to, the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can be adjusted as the various factors change over time.

In accordance with the present invention, it is provided a use of a compound of Formula (I) or a pharmaceutically acceptable salt thereof (e.g., tris(8-quinolinolato)gallium(III)) for the manufacture of a medicament for treating hematological cancer of myeloid origin. The medicament can be, e.g., in an oral or injectable form, e.g., suitable for intravenous, intradermal, or intramuscular administration. Injectable forms are generally known in the art, e.g., in buffered solution or suspension.

In accordance with another aspect of the present invention, a pharmaceutical kit is provided comprising in a container a unit dosage form of a compound of Formula (I) or a pharmaceutically acceptable salt thereof (e.g., tris(8-quinolinolato)gallium(III)), and optionally instructions for using the kit in accordance with the present invention, e.g., treating hematological cancer of myeloid origin, or treating refractory hematological cancer of myeloid origin. As will be apparent to a skilled artisan, the amount of a therapeutic compound in the unit dosage form is determined by the dosage to be used on a patient in accordance with the present invention. In the kit, a compound having a compound of Formula (I) or a pharmaceutically acceptable salt thereof (e.g., tris(8-quinolinolato)gallium(III)) can be used in a tablet form in an amount of, e.g., 1 mg.

EXAMPLE 1 (REFERENCE)

Cells have been expanded under appropriate culture conuitions until sufficient cells were available for the assay. Adherent cells were trypsinated. Cell count and viability of the cells were determined (Casy TT, Schärfe Systems). The Cells (viability >95%) were seeded in 100 μl of cell culture medium at the appropriate density of living cells into 96-well tissue culture plates. The cells were incubated at 37°C and 5% $CO_2$ for 24 hours.

100 μl of each dilution of tris(8-quinolinolato)gallium(III) ("drug") was added to the cells in triplicates. For a negative control, 100 μl of cell culture medium were added to three wells (100% value). For a positive control all cells were deadened with phenol (0% value). The cells were incubated with the drug for further 48 hours at 37°C and 5% $CO_2$.

XTT assays were performed on the above treated cells. XTT is a tetrazolium salt that can be cleaved by "succinate-tetrazolium reductase", a mitochondrial redox-system that is exclusively active in living cells. The cleavage results in a soluble formazan salt that can be quantified by colorimetric measuring at 450 nm. The intensity of the orange

color is directly linked to the number of living, metabolically active cells. A concentrated stock solution (1 mg/ml PBS) of XTT (Sigma-Aldrich) was prepared, aliquoted, filtered and stored at -20°C. 40 μl of the XTT-solution were added to each well of the assay plates and the cells were incubated with XTT for 8 hour at 37°C and 5% $CO_2$. The developed formazan was quantified in an absorbance micro plate reader (Genios, Tecan) at a measurement wavelength of 450 nm and a reference wavelength of 690 nm.

The tumor cell lines tested are summarized below in Table 1. Tris(8-quinolinolato)gallium(III) was effective in inhibiting cell growth and proliferation in all of the cell lines with $IC_{50}$ values all below 3.5 μM.

Table 1

| **Cell Line** | Description |
|---|---|
| CCRF-CEM | human T cell lymphoblast-like cell line |
| Jurkat | human T lymphocyte cell line |
| MOLT-4 | human acute lymphoblastic leukemia |

EXAMPLE 2

To test the activities of tris(8-quinolinolato)gallium(III) ("drug"), MTT assays were performed using selected hematological cancer cell lines. Cells were plated (2x$10^3$ cells in 100 μl/well) in 96-well plates and allowed to recover for 24 hours. The drug was added in another 100 μl growth medium and incubated with cultured cells for 3 hours before the cell culture medium was replaced to remove the drug. Cell death was measured 72 hours after the initial incubation by MTT assay following the manufacturer's recommendations (EZ4U, Biomedica, Vienna, Austria). The cell lines tested are summarized below in Table 2. Tris(8-quinolinolato)gallium(III) was effective in inducing cell death in all cell lines in Table 2 with $IC_{50}$ values ranging from about 1.8 μM to about 3.5 μM. Cells over-expressing MRP-1 or Pgp proteins and cells not over-expressing such MDR proteins were both tested, and there was no statistical difference in $IC_{50}$ value. That is, cells over-expressing MRP-1 or Pgp protein were not resistant to tris(8-quinolinolato)gallium(III). PGP/MRP-1-overexpression confers resistance to drugs such as vincristine, vinblastine, vinorelbine, taxol, docetaxel, etoposide, mitoxantrone, doxorubincin, epirubicin, topotecan, irinotecan, methotrexate, and imatinib etc. *See* Fojo & Menefee, Ann. Oncol., 18 (Supplement 5):v3-v8 (2007). Thus, tris(8-quinolinolato)gallium(III) should be effective in hematological cancer cells resistant to such drugs.

Table 2

| Cell Line | Description |
|---|---|
| HL-60 | Human acute promyelocytic leukemia cells |
| HL60/adr (mrp1 overex) | Human acute promyelocytic leukemia cells (over-expressing MRP1) |
| HL60/vinc (Pgp overex) | Human acute promyelocytic leukemia cells (resistant to vincaloids, over-expressing Pgp protein) |
| K562 | chronic myelocytic leukemia cell line |
| K562 (Pgp overex) | chronic myelocytic leukemia cell line (over-expressing Pgp protein) |

EXAMPLE 3 (REFERENCE)

To determine the growth inhibitory activity of tris(8-quinolinolato)gallium(III) and lenalidomide, a representative panel of human multiple myeloma tumor cell lines (OPM-2, RPML-8226, NCI-H929 and ARH-77) were tested with the compounds in anti-proliferation assays. Specifically, the human tumor cells were placed in a 96-well microculture plate at the appropriate density for 96 hours of total growth time. After 24 hours of incubation in a humidified incubator at 37 °C with 5% $CO_2$ and 95% air, serially diluted test agents in growth medium were added to each well. After 96 total hours of culture in a $CO_2$ incubator, the plates were processed with Cell Titer-Glo (Promega #G7571) according to manufacturer's instructions. Luminescence was detected using a Tecan GENios microplate reader. Percent inhibition of cell growth was calculated relative to untreated control wells. All tests were performed in duplicate at each concentration level.

The $IC_{50}$ value for the test agents was estimated using Prism 3.03 by curve-fitting the data using the following four parameter-logistic equation:

$$Y = \frac{Top - Bottom}{1 + \left( X / IC_{50} \right)^{n}} + Bottom$$

where *Top* is the maximal % of control absorbance, *Bottom* is the minimal % of control absorbance at the highest agent concentration, *Y* is the % of control absorbance, Xis the agent concentration, $IC_{50}$ is the concentration of agent that inhibits cell growth by 50% compared to the control cells, and *n* is the slope of the curve. The $IC_{50}$ data is summarized in Table 3 below:

Table 3

| Cell Line | Drug $IC_{50}$ ($\mu$M) | Lenalidomide $IC_{50}$ ($\mu$M) |
|---|---|---|
| OPM-2 | 0.74 | 5.83 |
| RPMI-8226 | 0.82 | Inactive |
| H929 | 1.57 | 5.28 |
| ARH-77 | 0.89 | Inactive |

EXAMPLE 4

**Activities of Tris(8-quinolinolato)gallium(III) in Human Leukemia Cells**

The human leukemia cell line MV4-11 cells were placed in a 96-well microculture plate (Costar white, flat bottom #3917) in a total volume of 90 $\mu$L/well. After 24 hours of incubation in a humidified incubator at 37°C with 5% $CO_2$ and 95% air, 10 $\mu$L of 10X, serially diluted tris(8-quinolinolato)gallium(III) in growth medium was added to each well. After 96 total hours of culture in a $CO_2$ incubator, the plated cells and Cell Titer-Glo (Promega #G7571) reagents were brought to room temperature to equilibrate for 30 minutes. 100 $\mu$L of Cell Titer-Glo® reagent was added to each well. The plate was shaken for 2 minutes and then left to equilibrate for 10 minutes before reading luminescence on the Tecan GENios microplate reader. Percent inhibition of cell growth was calculated relative to untreated control wells. All tests were performed in duplicate at each concentration level. The $IC_{50}$ value for the test agent was estimated using Prism 3.03 by curve-fitting the data using the following four parameter-logistic equation:

$$Y = \frac{Top - Bottom}{1 + \left( X / IC_{50} \right)^{n}} + Bottom$$

where *Top* is the maximal % of control absorbance, *Bottom* is the minimal % of control absorbance at the highest agent concentration, *Y is* the % of control absorbance, *X* is the agent concentration, *IC50* is the concentration of agent that inhibits cell growth by 50% compared to the control cells, and *n* is the slope of the curve. The compound tris(8-quinolinolato)gallium(III) had an $IC_{50}$ on MV4-11 of 1.44$\mu$M. It has been known that the MV4-11 cells are resistant to Ara-C, fludarabine, and doxorubicin. *See* Colado et al., Haematologica., 93(1):57-66 (2008); Scatena et al., Cancer Chemother. Pharmacol., 66(5):881-8 (2010). Thus, tris(8-quinolinolato)gallium(III) is active against leukemia cells resistant to Ara-C, fludarabine, and doxorubicin.

EXAMPLE 5 (REFERENCE)

In order to further explore the preclinical activity profile of tris(8-quinolinolato)gallium(III), its antiproliferative effects and cytotoxic potential in human malignant cell lines of lymphoma and leukemia were investigated. Further aims of the present study were the investigation of the ability of tris(8-quinolinolato)gallium(III) to overcome multiple drug resistance to conventional cancer therapeutics and to assess the major apoptosis signaling pathway triggered by this new agent.

**A. Methods and materials**

Tris(8-quinolinolato)gallium(III) was synthesized in high purity at the Institute of Inorganic Chemistry, University

of Vienna, Austria, according to the established procedure. The agent was dissolved in DMSO from Serva (Heidelberg, Germany) to give a 40 mM stock solution.

The following cells were used: BJAB (Burkitt like lymphoma) cells, mock and FADD transfected BJAB cells (BJAB FADD cells do not express the FADD protein, which activates the CD95 receptor dependent apoptotic pathway); Nalm-6 (human B cell precursor leukemia) cells; as well as vincristine- and daunorubicin-resistant Nalm-6 cells. The cells were subcultured every 3-4 days by dilution of the cells to a concentration of 1 $\times$ $10^5$/ml. All experiments were performed in RPMI 1640 medium (GIBCO, Invitrogen) supplemented with 10% heat inactivated fetal calf serum, 100 U/ml penicillin, 100 $\mu$g/ml streptomycin and 0.56 g/l L-glutamine. Twenty-four hours before the assay was setup, cells were cultured at a concentration of 3 x $10^5$/ml to attain standardized growth conditions. For apoptosis assays, the cells were then diluted to a concentration of 1 x $10^5$/ml immediately before addition of the different drugs.

Cytotoxicity of the different drugs was measured by the release of lactate dehydrogenase (LDH). After incubation with different concentrations of tris(8-quinolinolato)gallium(III) for 1 hour, LDH activity released by BJAB cells was measured in the cell culture supernatants using the Cytotoxicity Detection Kit from Boehringer Mannheim® (Mannheim, Germany). The supernatants were centrifuged at 1500 rpm for 5 min. 20 ul of cell-free supernatants were diluted with 80 $\mu$l phosphate-buffered saline (PBS), and 100 $\mu$l reaction mixture containing 2-[4-iodophenyl]-3-[4-nitrophenyl]-5-phenyltetrazolium chloride (INT), sodium lactate, NAD+ and diaphorase were added. Then, time-dependent formation of the reaction product was quantified photometrically at 490 nm. The maximum amount of LDH activity released by the cells was determined after lysis of the cells using 0.1% Triton X-100 in culture medium and set to represent 100% cell death.

Cell viability was determined by using the CASY® Cell Counter + Analyzer System of Innovatis (Bielefeld, Germany). Settings were specifically defined for the requirements of the cells used. With this system the cell concentration can be analyzed simultaneously in three different size ranges: thus cell debris, dead cells, and viable cells could be determined in one measurement. Cells were seeded at a density of 1 x $10^5$cells/ml and treated with different concentrations of [$Fe^{III}$(salophene)Cl]; non-treated cells served as controls. After a 24-hour incubation period, cells were re-suspended completely, and 100 $\mu$l of each well were diluted in 10 ml CASYton (ready-to-use isotonic saline solution) for immediate automated counting.

Apoptotic cell death was determined by a modified cell cycle-analysis, which detects DNA fragmentation on the single cell level. Cells were seeded at a density of 1 x $10^5$ cells/ml and treated with different concentrations of tris(8-quinolinolato)gallium(III). After a 72-hour incubation period at a temperature of 37 °C, cells were collected by centrifugation at 1500 rpm for 5 min, washed with PBS at 4 °C and fixed in PBS/2% (v/v) formaldehyde on ice for 30 minutes. After fixation, cells were pelleted, incubated with ethanol/PBS (2:1, v/v) for 15 minutes, pelleted and resuspended in PBS containing 40 $\mu$g/ml RNase. RNA was digested for 30 minutes at a temperature of 37 °C, after which the cells were pelleted again and finally resuspended in PBS containing 50 $\mu$g/ml propidium iodide. Nuclear DNA fragmentation was quantified by flow cytometric determination of hypodiploid DNA. Data were collected and analyzed using a FACScan instrument (Becton Dickinson, Heidelberg, Germany) equipped with CELL Quest software. Data are given in percent hypodiploidy (subG1), which reflects the number of apoptotic cells.

For Western blot analysis, cytosolic protein (15 mg) was loaded in each lane and was separated by sodium dodecylsulfate (SDS) PAGE. After blotting of proteins onto nitrocellulose membranes (Schleicher and Schuell, Dassel, Germany), the membrane was blocked for 1 h in PBST (PBS containing 0.05 % Tween-20) containing 3 % nonfat dry milk and incubated with primary antibody overnight at 4 °C. After the membrane had been washed three times in PBST, secondary antibody in PBST was applied for 1 h. Finally, the membrane was washed in PBST again and the ECL (enhanced chemiluminescence) system from Amersham Buchler (Braunschweig, Germany) was used to visualize the protein bands in question.

For the analysis of the differential expression of multiple genes involved in the different apoptosis pathways, apoptosis-specific RT2 profiler (polymerase chain reaction) PCR expression arrays (SuperArray PAHS-012; SABiosciences Corporation, Frederick, MD, USA) was used according to the manufacturer's instructions. Total RNA was extracted from BJAB cells treated with Titanocene Y (30 $\mu$M) for 8 hours, and RNAs were treated with DNase I (2 U/$\mu$l) to eliminate possible genomic DNA contamination. Total RNA (700 ng/$\mu$l) was then used as a template for the synthesis of a cDNA probe and subjected to quantitative real-time PCR SuperArray analysis according to the manufacturer's instructions using a LightCycler480 (Roche Diagnostics). The means of nine housekeeping genes were used to normalize the hybridization signals. Results were analyzed using SuperArray Analyser Software, and the data are given in -fold expression of the respective genes as compared with control cells incubated in vehicle-containing medium for 8 hours.

**B. Results**

1. Antiproliferative effects of tris(8-quinolinolato)gallium(III)

Unspecific cytotoxic effects of the agent such as necrosis could be excluded by determination of extracellular lactate dehydrogenase (LDH) via ELISA detection. After exposition of lymphoma cells (BJAB) to the agent for 1 hour

no significant LDH release could be observed, showing that the cell death was not caused by unspecific effects.

In order to assess the antiproliferative effects of tris(8-quinolinolato)gallium(III), BJAB lymphoma cells were exposed to various concentrations of the drug for 24 hours. The determination of cell viability and cell count were carried out with a CASY® Cell Counter and Analyzer System. As seen in **Figure 1,** tris(8-quinolinolato)gallium(III) inhibits tumor cell proliferation in a dose-dependent manner with a high potency, resulting in a steep concentration-effect curve with an $IC_{50}$ value slightly below 1 $\mu$M and a complete block of proliferation at concentrations $\geq$ 2 $\mu$M.

2. Tris(8-quinolinolato)gallium(III) induced apoptosis is mediated by a decrement of the mitochondrial membrane potential

The determination of the mitochondrial permeability transition via flow cytometric measurement reflects cells with decreased mitochondrial membrane potential, thus indicating that these cells undergo apoptosis via the mitochondrial intrinsic pathway. As it can be gauged from **Figure 2,** tris(8-quinolinolato)gallium(III) showed a dose-dependent increment of lymphoma cells (BJAB) with impaired mitochondrial permeability transition.

3. Induction of apoptosis in vitro

To quantify the induction of apoptosis triggered by tris(8-quinolinolato)gallium(III) we determined the DNA fragmentation (hypodiploidy) as a characteristic effect of apoptotic cell death. The procedure was conducted by flow cytometric measurement of hypodiploid DNA after incubating lymphoma cells (BJAB) for 72 hours with the agent. Extensive apoptosis induction could be reached even in low concentrations of the agent ($AC_{50}$: ~1 $\mu$M), seen in **Figure 3.**

4. Tris(8-quinolinolato)gallium(III) overcomes resistance to vincristine

Multidrug resistance (MDR) is a phenomenon of simultaneous resistance to unrelated chemotherapeutic drugs. P-glycoprotein is member of the ATP-binding cassette (ABC) transporter family and is known to cause MDR by its overexpression and to mediate active transport of toxic compound out of the cell. Tumor cells potentially use various ABC transporters to build up multidrug resistances, thus implying an immediate obstacle to therapeutic treatment of malignant diseases. *See* Fojo & Menefee, Ann. Oncol., 18 Suppl 5:v3-8 (2007). Anthracyclines such as daunorubicin and Vinca alkaloids such as vincristine are potent agents used in cytotoxic chemotherapy. However, both classes of compounds are capable of inducing multidrug resistance. A significant overexpression of P-gp could be verified via FACS analysis in vincristine and daunorubicin resistant Nalm-6 cells, which are additionally resistant to fludarabine and paclitaxel. After 72 hours of incubation of both resistant cell lines with tris(8-quinolinolato)gallium(III), DNA fragmentation was measured. **Figure 4** demonstrates that tris(8-quinolinolato)gallium(III) is able to induce higher apoptotic amounts in the resistant cells compared to the control cells and thus overcomes multidrug resistance.

5. Tris(8-quinolinolato)gallium(III) induces apoptosis via the intrinsic pathway

The intrinsic pathway is characterized by a loss of mitochondrial membrane potential, as it could be shown in **Figure 2.** To underline the suggestion that tris(8-quinolinolato)gallium(III) induces apoptosis via the intrinsic pathway, the involvement of CD95/Fas death receptor-mediated apoptosis could be excluded. For that purpose a cellular model system was used consisting of BJAB cells overexpressing a dominant-negative FADD mutant (BJAB FADDdn) and BJAB control cells (BJAB mock). A determination of apoptotic cells via DNA fragmentation resulted in similar apoptotic rates **(Figure 5).** Thus, we conclude that tris(8-quinolinolato)gallium(III) induced apoptosis in BJAB cells occurs independently of CD95/Fas and FADD signaling.

Further evidence for apoptosis via the intrinsic pathway was obtained by investigating the consecutive activation of caspase-9 and -3 by Western blot analysis. Both are crucial effector proteins of apoptotic signal transduction and execution. The activation of both caspases by proteolytic cleavage was determined after treatment of BJAB cells with tris(8-quinolinolato)gallium(III). As shown in figure 6, tris(8-quinolinolato)gallium(III) induces processing of procaspase-3 (p32) and procaspase-9 (p47), resulting in active subunits of both caspases (p17, p18; p37). Equal protein loading was confirmed by ß-actin.

6. Tris(8-quinolinolato)gallium(III)-induced apoptosis leads to an upregulation of caspase-5 and Harakiri

Changes of transcript levels of apoptosis relevant genes were analyzed in RNA isolated from BJAB cells after 8 hours of incubation with 2.5 $\mu$M tris(8-quinolinolato)gallium(III). Via real-time PCR we detected a significant elevation of the caspase-5 (118-fold upregulation) and Harakiri (29-fold upregulation) gene expression. Harakiri (Hrk) is a pro-apoptotic Bcl-2 homology domain 3-only protein of the Bcl-2 family. These results indicate a high influence of tris(8-quinolinolato)gallium(III) treatment on the transcription of both pro-apoptotic genes.

**C. Discussion**

Apoptosis is a morphologically distinct form of programmed cell death that plays a major role during development, homeostasis and in various diseases including cancer. Since the appearance of malignancies is due to deregulated proliferation and inability of cells to undergo apoptosis, potential anticancer drugs with the capability to inhibit proliferation and induce apoptosis in tumor cells are urgently needed. Vincristine, a Vinca alkaloid and mitotic inhibitor, as well as daunorubicin, an anthracycline with DNA-damaging property, are among the most important antitumor drugs available and used exclusively for the treatment of leukemia.

The results here convincingly demonstrate that the investigated novel agent tris(8-quinolinolato)gallium(III) strongly inhibits the proliferation of lymphoma cells (BJAB) up to 100% and leads to a concentration-dependent induction of apoptosis in cells of lymphoma (BJAB). The quantification of tris(8-quinolinolato)gallium(III) induced apoptosis was measured by DNA fragmentation via FACS analysis after 72 hours and displayed high apoptotic efficiency at low micromolar concentrations ($LC_{50}$ in BJAB cells: ~1 $\mu$M). Necrotic cell death, characterized by the early release of intracellular lactate dehydrogenase (LDH), could be excluded, as the effects seen in the LDH assay after 1-hour incubation were negligible. The considerable potential of tris(8-quinolinolato)gallium(III) as a cytotoxic agent is further underlined by gene expression profiling data of various apoptosis relevant genes, WO 2011/100642 PCT/US2011/024662 obtained via real-time PCR, which revealed an upregulation of the pro-apoptotic genes caspase-5 (118-fold) and Harakiri (29-fold). Moreover, it has been demonstrated that tris(8-quinolinolato)gallium(III) significantly induces apoptosis via the intrinsic mitochondrial pathway in a caspase dependent manner. These observations could be indicated by the loss of mitochondrial membrane potential, FADD independence and the detection of caspase-3 and caspase-9 in BJAB cells, respectively. Anticancer treatment using cytotoxic drugs is considered to mediate cell death by activating caspases, proteolytic enzymes that act as key elements and serve as main effectors of the apoptosis program.

Failure of therapeutic treatment may due to the development of multidrug resistance (MDR), a mechanism which is responsible for the upregulation of membrane transporters such as P-glycoprotein (P-gp) that is involved in the efflux of cytotoxic drugs from tumor cells. Since the treatment of children with acute lymphoblastic leukemia (ALL) is based on P-gp-dependent cytostatic drugs and P-gp expression is considered to correlate with poor prognosis and a high probability of relapse, vincristine- and daunorubicin-resistant Nalm-6 cells were investigated, which are additionally resistant to fludarabine and paclitaxel, respectively, and overexpress P-gp. Both resistant cell lines revealed higher sensitivity to the treatment with tris(8-quinolinolato)gallium(III) compared to the control cells. This finding clearly shows that the agent is capable to overcome multidrug-resistance in the cells.

The data suggest here that tris(8-quinolinolato)gallium(III) is a promising, new anti-cancer agent with anti-leukemic properties which are maintained even in cell models that are resistant to conventional forms of chemotherapy because of an overexpression of P-gp.

EXAMPLE 6 (REFERENCE)

To determine the antiproliferative activity of tris(8-quinolinolato)gallium(III) in human lymphoma tumor cell lines, anti-proliferation assays were conducted in the DoHH2, Granta 519, and WSU-DLCL2 cell lines. The DoHH2 Human EBV-negative B Cell Lymphoma cells were seeded with 5,000 cells/well and grown in RPMI1640 medium containing 20% FBS, and 2 mM L-Glutamine. The Granta 519 Human Mantle Cell Lymphoma cells were seeded with 10,000 cells/well and grown in DMEM medium containing 10% FBS, and 2 mM L-Glutamine. The WSU-DLCL2 Human B Cell Lymphoma cells were seeded with 5,000 cells/well and grown in RPMI1640 medium containing 10% FBS, and 2mM L-Glutamine. Specifically, the human tumor cells were placed in a 96-well microculture plate at the appropriate density for 96 hours of total growth time. After 24 hours of incubation in a humidified incubator at 37 °C with 5% $CO_2$ and 95% air, serially diluted test agents in growth medium were added to each well. After 96 total hours of culture in a $CO_2$ incubator, the plates were processed with Cell Titer-Glo (Promega #G7571) according to manufacturer's instructions. Luminescence was detected using a Tecan GENios microplate reader. Percent inhibition of cell growth was calculated relative to untreated control wells. All tests were performed in duplicate at each concentration level. The $IC_{50}$ value for the test agents was estimated using Prism 3.03 by curve-fitting the data using the four parameter-logistic equation as described in Example 4 above. The compound tris(8-quinolinolato)gallium(III) had an $IC_{50}$ of 0.213 $\mu$M in DoHH2 **(Figure 7**), 2.9 $\mu$M in Granta 519 **(Figure 8),** and 1.47 $\mu$M in WSU-DLCL2 **(Figure 9).** It is known that WSU-DLCL2 cells are resistant to the CHOP (cyclophosphamide, adriamycin, vincristine, prednisone) regimen. *See* Levi et al., Cancer Chemother. Pharmacol., (published online August 31, 2010). Thus, tris(8-quinolinolato)gallium(III) is also active against lymphoma cells resistant to drugs such as cyclophosphamide, adriamycin, vincristine, prednisone.

## Claims

1. A compound of Formula (I)

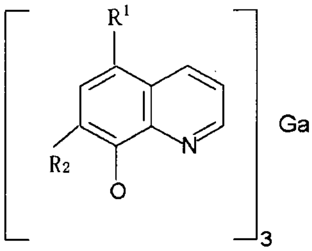

(I)

wherein $R^1$ represents hydrogen, a halogen or a sulfono group $SO_3M$, in which M is a metal ion, and $R^2$ represents hydrogen, or $R^1$ is Cl and $R^2$ is I, or a pharmaceutically acceptable salt thereof for use in the treatment of hematological cancer, wherein said hematological cancer is of myeloid origin.

2. Use of a compound as defined in Claim 1 or a pharmaceutically acceptable salt thereof for the manufacturing of a medicament for treating hematological cancer, wherein said hematological cancer is of myeloid origin.

3. The compound for use of Claim 1 or the use of Claim 2, wherein said compound is tris(8-quinolinolato)gallium(III).

4. The compound for use of Claim 1 or 3 or the use of Claim 2 or 3, wherein said hematological cancer is myelogenous leukemia.

5. The compound for use of any one of Claims 1, 3 and 4 or the use of any one of Claims 2 to 4, wherein said hematological cancer is not acute promyelocytic leukemia.

6. The compound for use of any one of Claims 1 and 3 to 5 or the use of any one of Claims 2 to 5, wherein said hematological cancer is selected from acute myeloblastic leukemia, acute megakaryoblastic leukemia, chronic myelogenous leukemia, acute monocytic leukemia, myelodysplastic syndromes (MDS), or myeloproliferative diseases.

7. The compound for use of any one of Claims 1 and 3 to 6 or the use of any one of Claims 2 to 6, wherein said hematological cancer is a refractory hematological cancer.

8. The compound for use of Claim 7 or the use of Claim 7, wherein said hematological cancer is refractory to a treatment comprising one or more drugs selected from the group consisting of vinca alkaloids, anthracyclines, anthracenediones, epipodophyllotoxins, camptothecins, lenalidomide, thalidomide, cytarabine and fludarabine.

9. The compound for use of Claim 7 or the use of Claim 7, wherein said hematological cancer is previously treated with a camptothecin drug (e.g., topotecan).

10. The compound for use of Claim 7 or the use of Claim 7, wherein said hematological cancer is chronic myelogenous leukemia (CML) previously treated with a PDGF-Rβ inhibitor (e.g., imatinib).

11. The compound for use of Claim 7 or the use of Claim 7, wherein said hematological cancer is acute myeloid leukemia previously treated with cytarabine.

12. The compound for use of Claim 7 or the use of Claim 7, wherein said hematological cancer is acute myeloid leukemia (AML) previously treated with fludarabine.

13. The compound for use of Claim 7, wherein said hematological cancer is MDS previously treated with lenalidomide.

**Patentansprüche**

1. Verbindung der Formel (I)

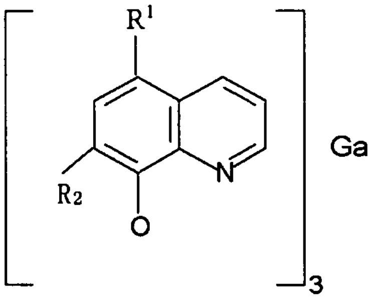

(I)

wobei $R^1$ Wasserstoff, ein Halogen oder eine Sulfonogruppe $SO_3M$ ist, worin M ein Metallion ist, und $R^2$ für Wasserstoff steht, oder $R^1$ Cl ist und $R^2$ I ist, oder ein pharmazeutisch verträgliches Salz davon, zur Verwendung bei der Herstellung eines Medikaments zur Behandlung von Blutkrebs, wobei der Blutkrebs myeloiden Ursprungs ist.

2. Verwendung einer Verbindung wie in Anspruch 1 definiert, oder eines pharmazeutisch verträglichen Salzes davon, zur Herstellung eines Medikaments zur Behandlung von Blutkrebs, wobei der Blutkrebs myeloiden Ursprungs ist.

3. Verbindung zur Verwendung nach Anspruch 1 oder Verwendung nach Anspruch 2, wobei die Verbindung Tris(8-chinolinolato)gallium(III) ist.

4. Verbindung zur Verwendung nach Anspruch 1 oder 3 oder Verwendung nach Anspruch 2 oder 3, wobei der Blutkrebs myelogene Leukämie ist.

5. Verbindung zur Verwendung nach einem der Ansprüche 1, 3 und 4 oder Verwendung nach einem der Ansprüche 2 bis 4, wobei der Blutkrebs keine akute Promyelozytenleukämie ist.

6. Verbindung zur Verwendung nach einem der Ansprüche 1 und 3 bis 5 oder Verwendung nach einem der Ansprüche 2 bis 5, wobei der Blutkrebs ausgewählt ist aus: akute myeloblastische Leukämie, akute megakaryoblastische Leukämie, chronische myelogene Leukämie, akute monozytische Leukämie, myelodysplastische Syndrome (MDS) oder myeloproliferative Erkrankungen.

7. Verbindung zur Verwendung nach einem der Ansprüche 1 und 3 bis 6 oder Verwendung nach einem der Ansprüche 2 bis 6, wobei der Blutkrebs refraktärer Blutkrebs ist.

8. Verbindung zur Verwendung nach Anspruch 7 oder Verwendung nach Anspruch 7, wobei der Blutkrebs refraktär bezüglich einer Behandlung ist umfassend ein oder mehrere Arzneimittel ausgewählt aus der Gruppe bestehend aus: Vinca-Alkaloide, Anthracycline, Anthracendione, Epipodophyllotoxine, Camptothecine, Lenalidomid, Thalidomid, Cytarabin und Fludarabin.

9. Verbindung zur Verwendung nach Anspruch 7 oder Verwendung nach Anspruch 7, wobei der Blutkrebs vorher mit einem Camptothecin-Medikament (z.B. Topotecan) behandelt wurde.

10. Verbindung zur Verwendung nach Anspruch 7 oder Verwendung nach Anspruch 7, wobei der Blutkrebs chronische myelogene Leukämie (CML) ist, die zuvor mit einem PDGF-Rβ Inhibitor behandelt wurde (z.B. Imatinib).

11. Verbindung zur Verwendung nach Anspruch 7 oder Verwendung nach Anspruch 7, wobei der Blutkrebs akute myeloide Leukämie ist, die zuvor mit Cytarabin behandelt wurde.

12. Verbindung zur Verwendung nach Anspruch 7 oder Verwendung nach Anspruch 7, wobei der Blutkrebs akute myeloide Leukämie (AML) ist, die zuvor mit Fludarabin behandelt wurde.

13. Verbindung zur Verwendung nach Anspruch 7, wobei der Blutkrebs MDS ist, das zuvor mit Lenalidomid behandelt

wurde.

**Revendications**

1. Composé de Formule (I)

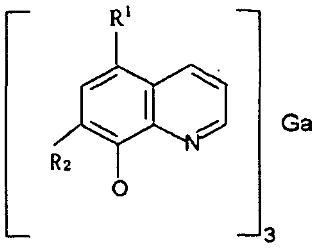

(I)

dans laquelle $R^1$ représente un atome d'hydrogène, un atome d'halogène ou un groupe sulfono $SO_3M$, M étant un ion métallique, et $R^2$ représente un atome d'hydrogène, ou $R^1$ est Cl et $R^2$ est I, ou un sel pharmaceutiquement acceptable de celui-ci destiné à être utilisé dans le traitement d'un cancer hématologique, dans lequel ledit cancer hématologique est d'origine myéloïde.

2. Utilisation d'un composé selon la revendication 1 ou d'un sel pharmaceutiquement acceptable de celui-ci dans la fabrication d'un médicament destiné à traiter un cancer hématologique, dans laquelle ledit cancer hématologique est d'origine myéloïde.

3. Composé pour son utilisation selon la revendication 1 ou utilisation selon la revendication 2, dans lequel ledit composé est un tris(8-quinolinolato)gallium (III).

4. Composé pour son utilisation selon la revendication 1 ou 3 ou utilisation selon la revendication 2 ou 3, dans lequel ledit cancer hématologique est la leucémie myélogène.

5. Composé pour son utilisation selon l'une quelconque des revendications 1, 3 et 4 ou utilisation selon l'une quelconque des revendications 2 à 4, dans lequel ledit cancer hématologique n'est pas la leucémie aiguë promyélocytaire.

6. Composé pour son utilisation selon l'une quelconque des revendications 1 et 3 à 5 ou utilisation selon l'une quelconque des revendications 2 à 5, dans lequel ledit cancer hématologique est choisi parmi la leucémie aiguë myéloblastique, la leucémie aiguë mégacaryoblastique, la leucémie myélogène chronique, la leucémie aiguë monocytaire, les syndromes myélodysplasiques (MDS), ou les maladies myéloprolifératives.

7. Composé pour son utilisation selon l'une quelconque des revendications 1 et 3 à 6 ou utilisation selon l'une quelconque des revendications 2 à 6, dans lequel ledit cancer hématologique est un cancer hématologique réfractaire.

8. Composé pour son utilisation selon la revendication 7 ou utilisation selon la revendication 7, dans lequel ledit cancer hématologique est réfractaire à un traitement comprenant un ou plusieurs médicaments choisis dans le groupe constitué par les vinca-alcaloïdes, les anthracyclines, les anthracènediones, les épipodophyllotoxines, les camptothécines, le lénalidomide, le thalidomide, la cytarabine et la fludarabine.

9. Composé pour son utilisation selon la revendication 7 ou utilisation selon la revendication 7, dans lequel ledit cancer hématologique est préalablement traité avec un médicament à base de camptothécine (p. ex., topotécan).

10. Composé pour son utilisation selon la revendication 7 ou utilisation selon la revendication 7, dans lequel ledit cancer hématologique est la leucémie myélogène chronique (CML) préalablement traitée avec un inhibiteur de PDGF-Rβ (p. ex., imatinib).

11. Composé pour son utilisation selon la revendication 7 ou utilisation selon la revendication 7, dans lequel ledit cancer hématologique est la leucémie aiguë myéloïde préalablement traitée à la cytarabine.

**12.** Composé pour son utilisation selon la revendication 7 ou utilisation selon la revendication 7, dans lequel ledit cancer hématologique est la leucémie aiguë myéloïde (AML) préalablement traitée à la fludarabine.

**13.** Composé pour son utilisation selon la revendication 7, dans lequel ledit cancer hématologique est un MDS préalablement traité au lénalidomide.

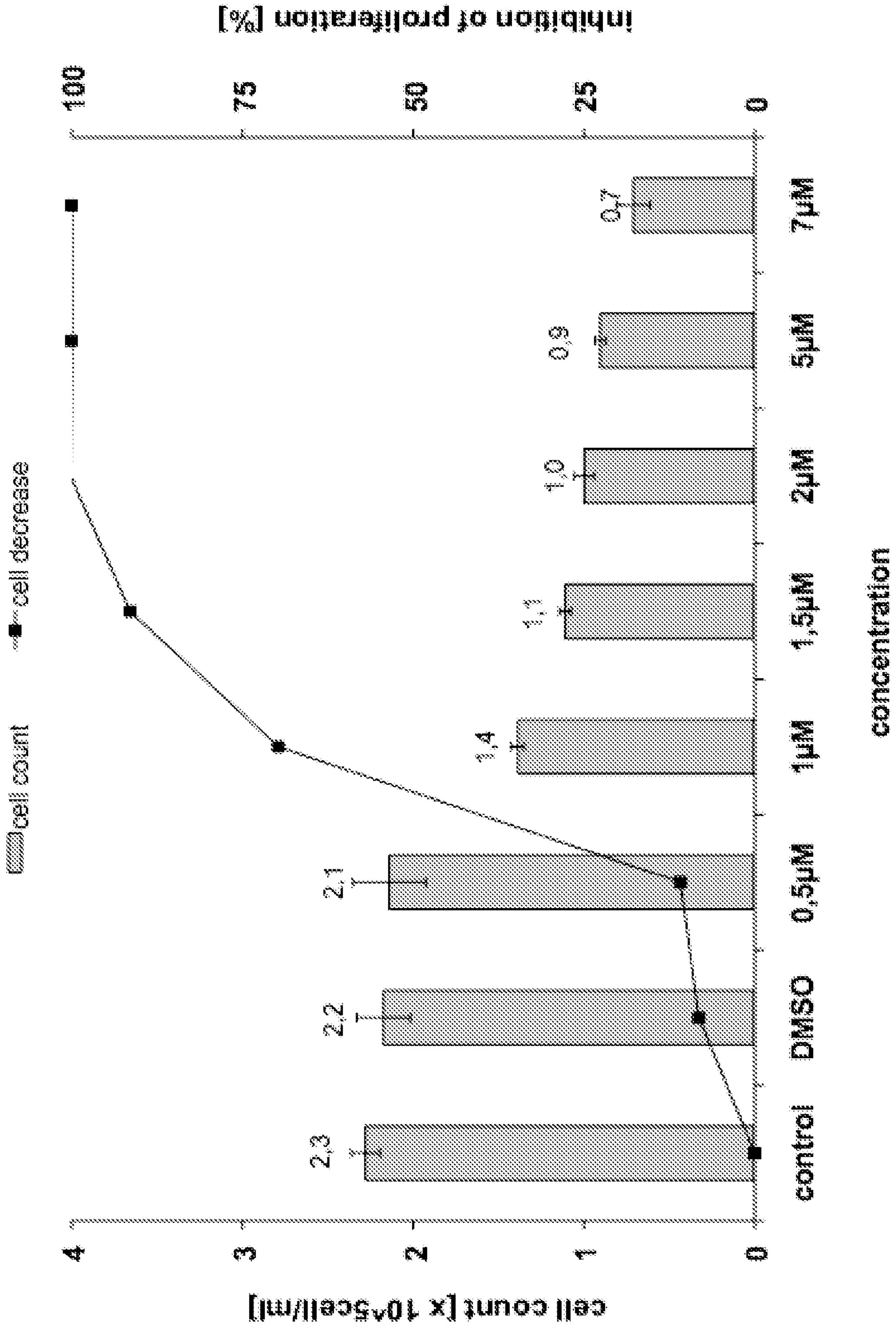

cell count
cell decrease
cell count [x 10^5cell/ml]
inhibition of proliferation [%]
concentration
control
DMSO
0,5µM
1µM
1,5µM
2µM
5µM
7µM
2,3
2,2
2,1
1,4
1,1
1,0
0,9
0,7
0
1
2
3
4
0
25
50
75
100


Figure 1

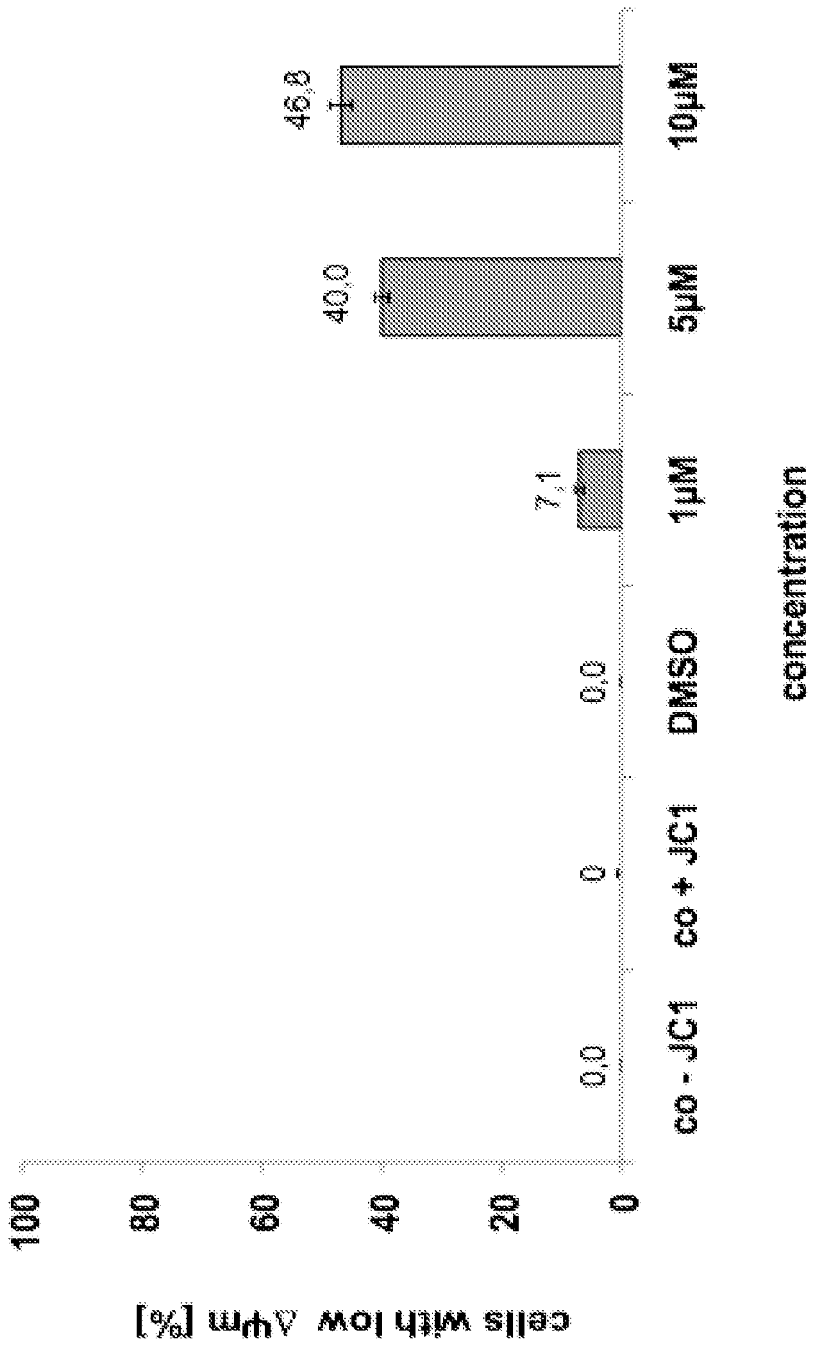

cells with low ΔΨm [%]
100
80
60
40
20
0
0,0
0
0,0
7,1
40,0
46,8
co - JC1
co + JC1
DMSO
1µM
5µM
10µM
concentration


Figure 2

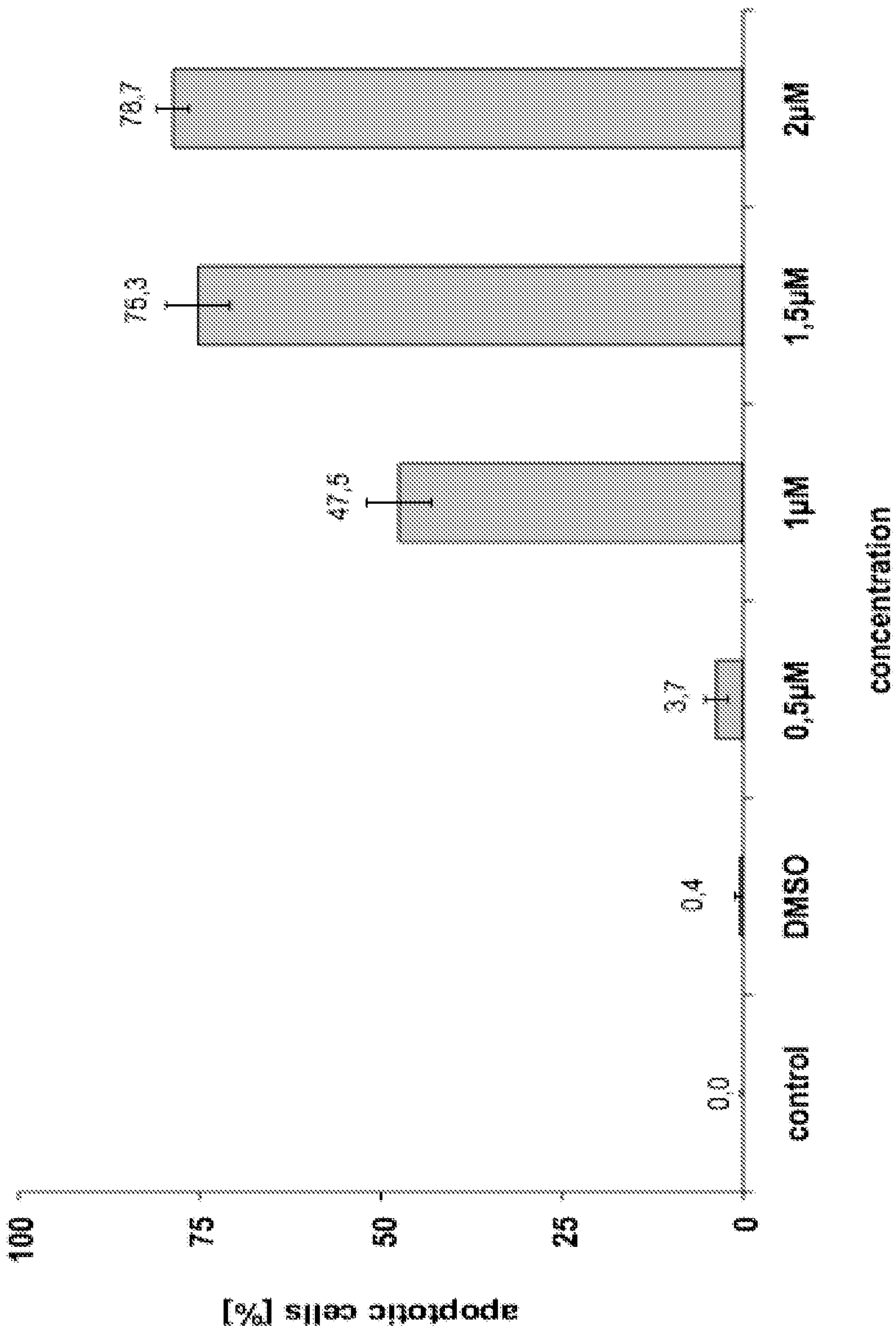
apoptotic cells [%]
100
75
50
25
0
0,0
0,4
3,7
47,5
75,3
78,7
control
DMSO
0,5µM
1µM
1,5µM
2µM
concentration

Figure 3

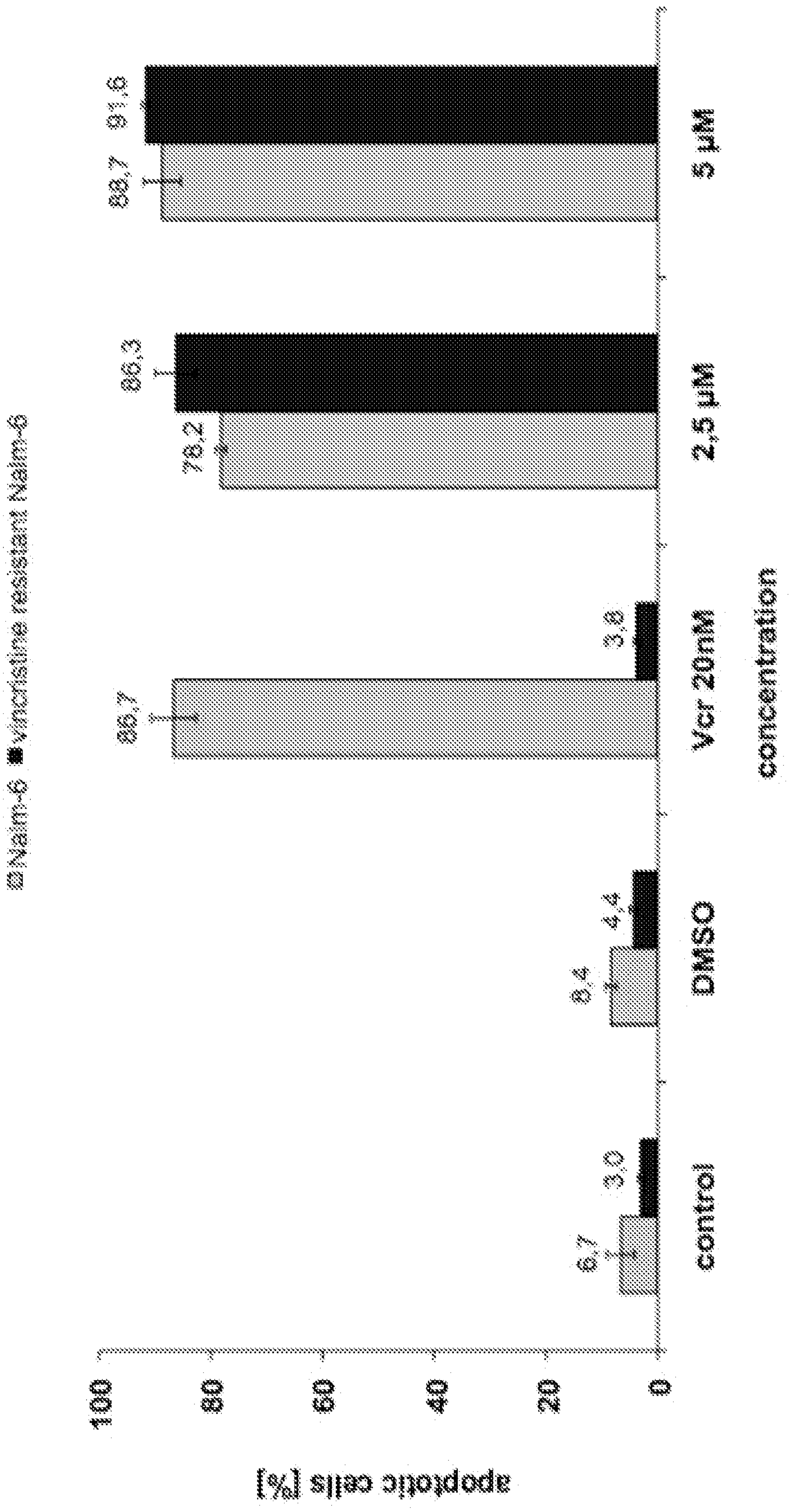
Nalm-6
vincristine resistant Nalm-6
apoptotic cells [%]
100
80
60
40
20
0
6,7
3,0
8,4
4,4
86,7
3,8
78,2
86,3
88,7
91,6
control
DMSO
Vcr 20nM
2,5 µM
5 µM
concentration

Figure 4A

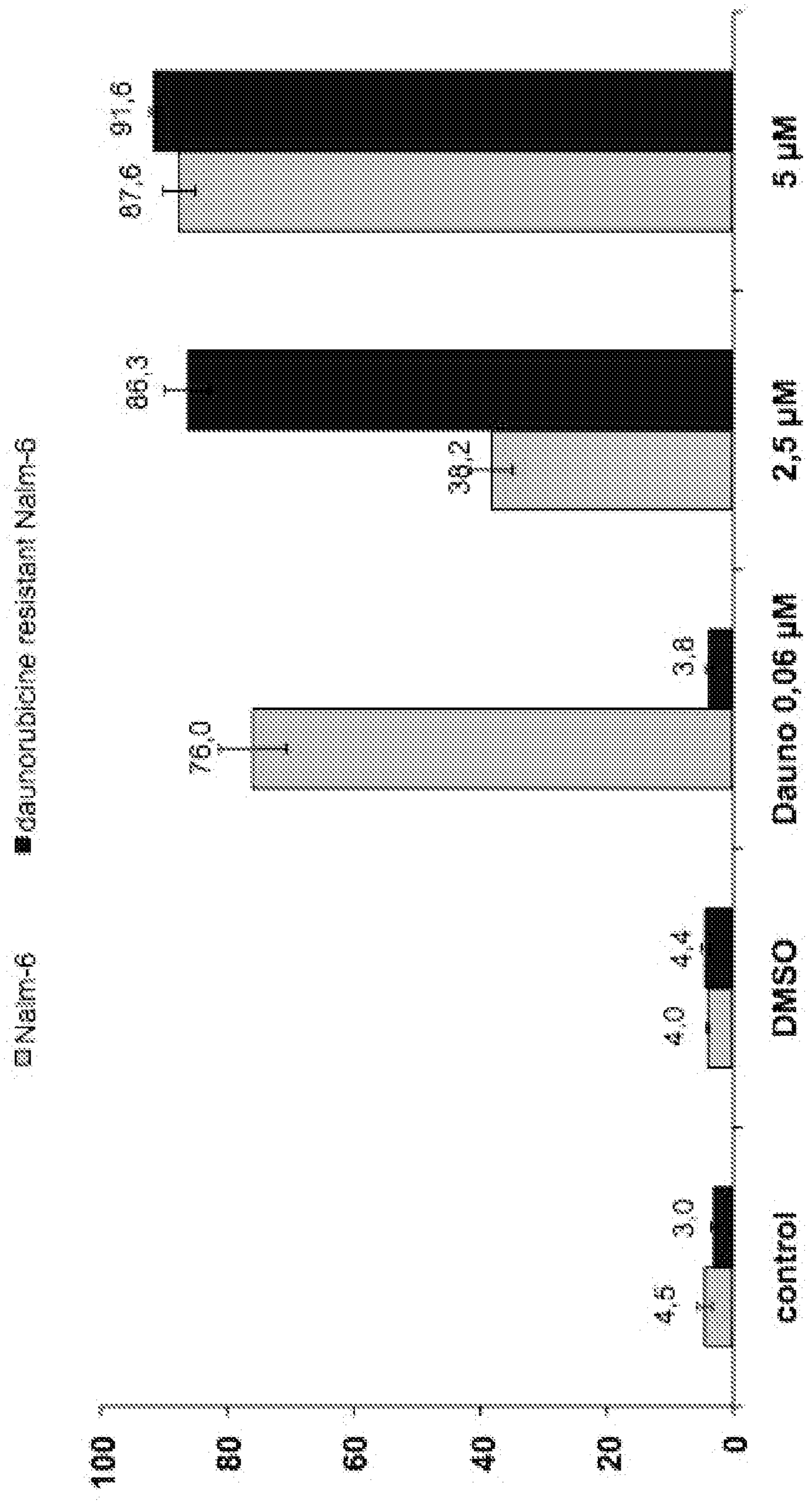
Naim-6
daunorubicine resistant Naim-6
100
80
60
40
20
0
control
DMSO
Dauno 0,06 µM
2,5 µM
5 µM
4,5
3,0
4,0
4,4
76,0
3,8
38,2
86,3
87,6
91,6

Figure 4B

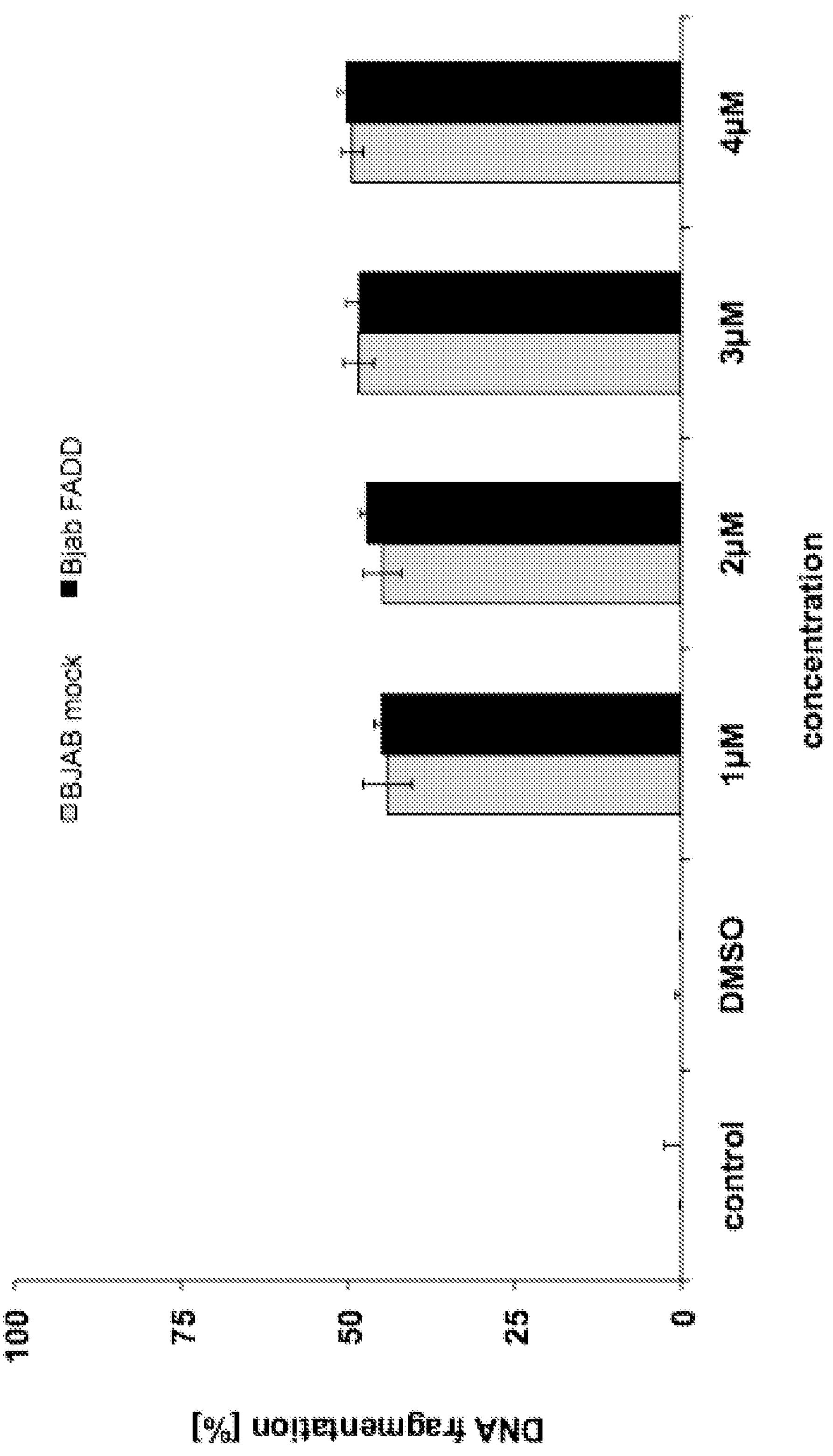
BJAB mock
Bjab FADD
DNA fragmentation [%]
100
75
50
25
0
control
DMSO
1µM
2µM
3µM
4µM
concentration

Figure 5

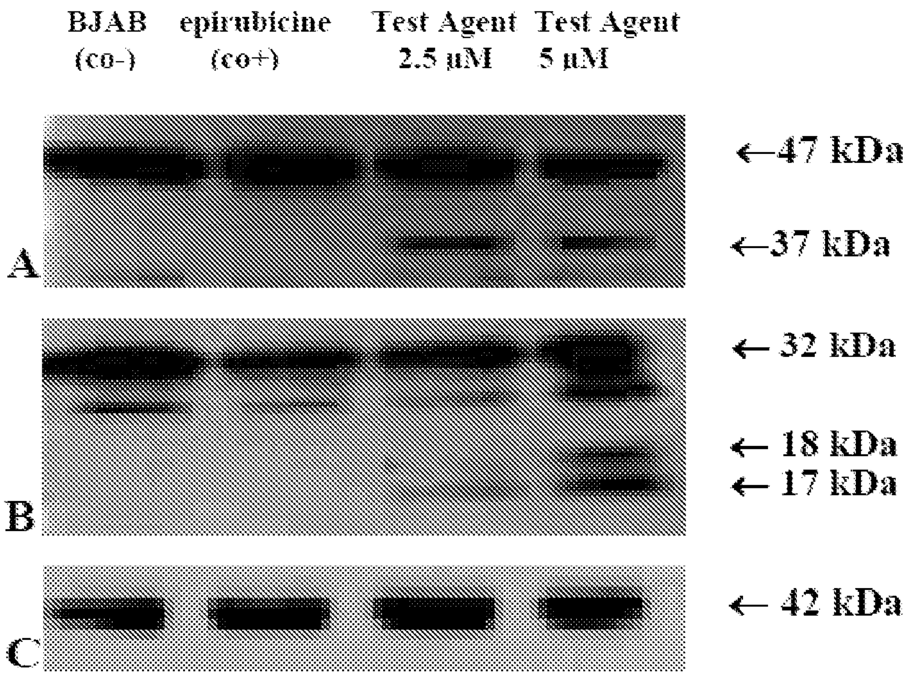
BJAB (co-)
epirubicine (co+)
Test Agent 2.5 µM
Test Agent 5 µM
←47 kDa
←37 kDa
A
← 32 kDa
← 18 kDa
← 17 kDa
B
← 42 kDa
C

Figure 6

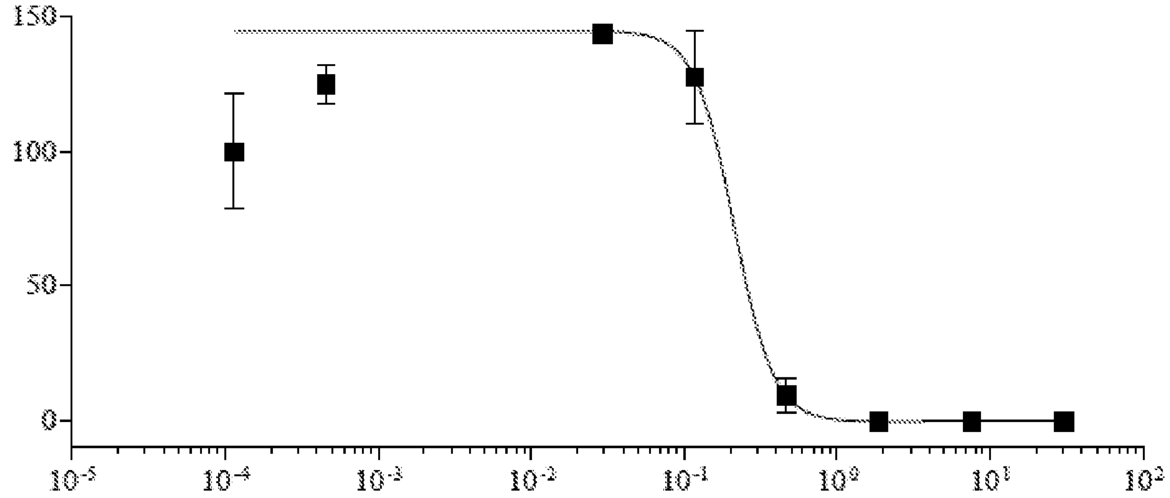


Figure 7

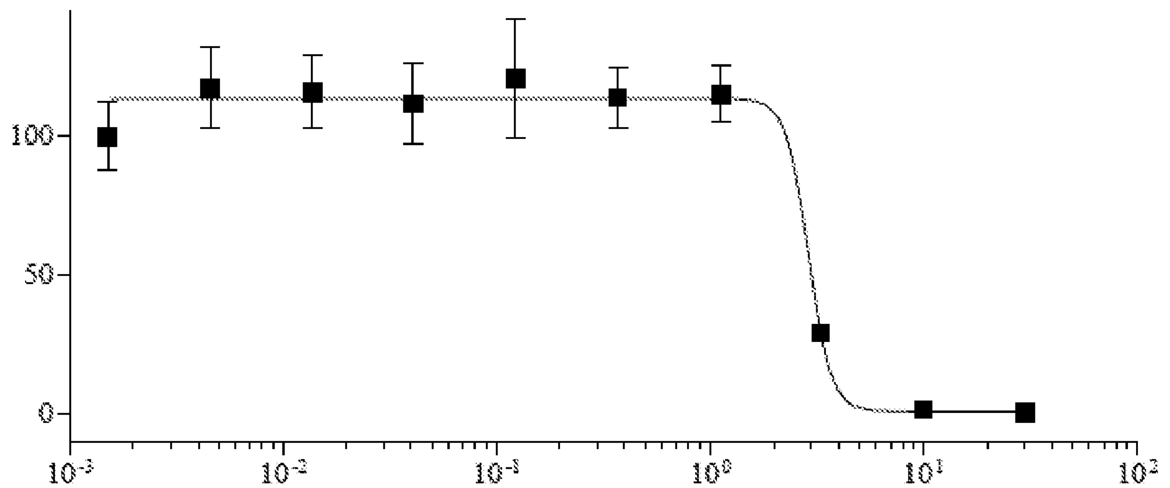


Figure 8

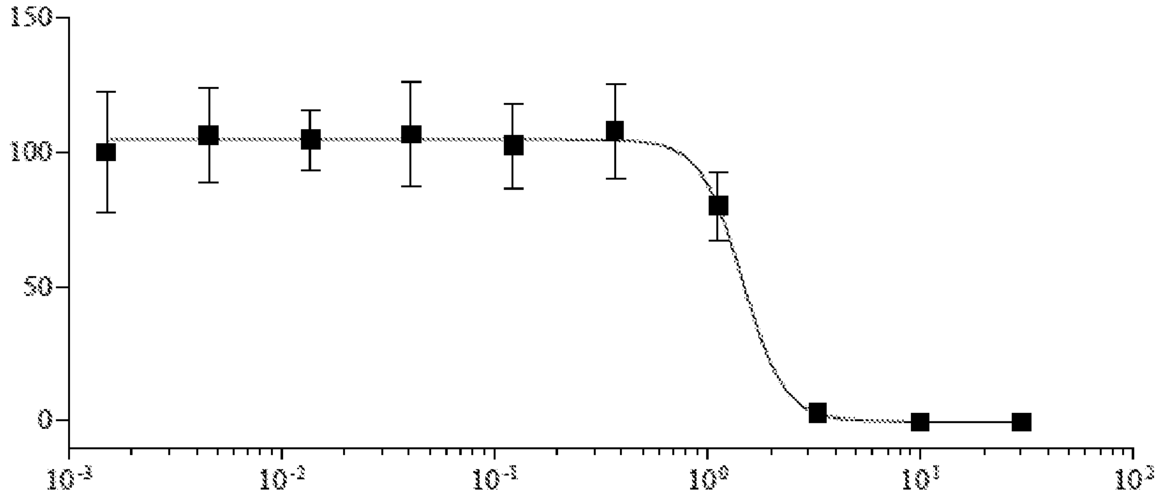


Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090137620 A
- WO 2011100642 A
- US 2011024662 W


### Non-patent literature cited in the description


- **JONKHOFF AR et al.** *Leuk Res.,* 1995, vol. 19 (3), 169-174
- **THERASSE et al.** *J. Natl. Cancer Inst.,* 2000, vol. 92, 205-216
- **FOJO ; MENEFEE.** *Ann. Oncol.,* 2007, vol. 18 (5), v3-v8
- **COLADO et al.** *Haematologica,* 2008, vol. 93 (1), 57-66
- **SCATENA et al.** *Cancer Chemother. Pharmacol.,* 2010, vol. 66 (5), 881-8
- **LEVI et al.** *Cancer Chemother. Pharmacol.,* 31 August 2010